# EUROPÄISCHE PATENTANMELDUNG

(11) **EP 1 333 279 A2**
(43) Veröffentlichungstag der Anmeldung: **06.08.2003**
(21) Anmeldenummer: 03001240.5
(22) Anmeldetag: 21.01.2003
(51) Int. Cl.: G01N 33/50

(54) **Verfahren zur Durchführung von elektrischen Messungen an biologischen Membrankörpern**

(30) Priorität: 31.01.2002 DE 10203686
(71) Anmelder: Bayer Aktiengesellschaft, 51368 Leverkusen (DE)
(72) Erfinder: Lison, Frank, Dr., 40764 Langenfeld (DE); Methfessel, Christoph, Dr., 42327 Wuppertal (DE); Schnizler, Katrin, Dr., 51061 Köln (DE)

(57) **Zusammenfassung**

Die vorliegende Erfindung betrifft ein Verfahren zur Durchführung elektrischer Messungen an biologischen Membrankörpern, die sich auf einem Träger befinden. Der für die Messung benötigte elektrisch leitende Zugang zum biologischen Membrankörper wird in situ durch intensive Lichtstrahlen erzeugt.

## Beschreibung

Die vorliegende Erfindung betrifft ein Verfahren zur Durchführung elektrischer Messungen an biologischen Membrankörpern, die sich auf einem Träger befinden. Der für die Messung benötigte elektrisch leitende Zugang zum biologischen Membrankörper wird in situ durch intensive Lichtstrahlen erzeugt.

Ionenkanäle und Rezeptoren gewinnen als Wirkstofftargets der pharmazeutischen Industrie, des Pflanzenschutzes und der Tiergesundheit zunehmend an Bedeutung. Besonders durch die Entschlüsselung der Genome des Menschen und anderer Organismen stehen der Wirkstoffforschung eine Vielzahl neuer, potentieller Wirkstofftargets zur Verfügung. In vielen Fällen sind dies neuartige lonenkanäle oder neue Untereinheiten bereits bekannter lonenkanäle. Für das Auffinden von Wirkstoffen, die Ionenkanäle modulieren, sind sogenannte elektrophysiologische Messungen unerlässlich. Dabei wird der durch die Ionenkanäle fließende Strom elektrisch geladener Teilchen direkt gemessen.

Stand der Technik bei der Untersuchung von Ionenkanälen in Zellmembranen ist heute das Patch-Clamp-Verfahren, das Mitte der 70'er Jahre von Neher und Sakmann entwickelt wurde (O. P. Hamill et al, Pflügers Arch. **391,** 85 (1981)). Mit dieser Methode konnte erstmals der Strom durch einzelne Ionenkanäle gemessen werden, zum anderen konnten nun auch kleinere Zellen elektrophysiologisch charakterisiert werden. Im Gegensatz zu den bis dato bekannten Verfahren für große Zellen, bei denen eine mit einem Elektrolyt gefüllte Glaskapillare oder Pipette in die Zelle einsticht, wird bei der Patch-Clamp Technik die Glaskapillare vorsichtig auf die Zellmembran aufgesetzt und diese leicht angesaugt. Dabei bildet sich das sogenannte Gigaseal, eine äußerst hochohmige, elektrisch dichte Verbindung zwischen der Pipettenspitze und der Zellmembran. Dies isoliert einen kleinen Membranfleck, den "Patch", vom Rest der Zelloberfläche und erlaubt damit, einzelne Ionenkanäle in diesem Patch zu beobachten. Durch einen Unterdruckpuls oder einen elektrischen Impuls oder geeignete chemische Substanzen kann der unter der Pipette isolierte Patch der Zellmembran zerstört werden und so ein elektrisch hochwertiger Zugang zum Zellinneren etabliert werden. Ein elektrisch hochwertiger Zugang ist eine niederohmige (< 20 Megaohm) leitende Verbindung zwischen dem Inneren der Pipette und dem Inneren der Zelle bei vernachlässigbarem (im oberen Megaohmbereich, bevorzugt im Gigaohmbereich) Parallelwiderstand zwischen dem Inneren der Pipette und der die Zelle umgebenden Elektrolytlösung. Dabei wird die Zelle ansonsten nicht beschädigt oder zerstört. In dieser sogenannten Ganzzellableitung wird die Aktivität von Ionenkanälen in der gesamten Membran der Zelle gleichzeitig beobachtet. Dank der hohen Qualität der elektrischen Ableitung kann man Ströme bis auf wenige Pikoampere bei Messzeiten von wenigen Millisekunden präzise ableiten und erhält so ein nahezu perfektes, detailliertes Bild der Aktivität von Ionenkanälen unter dem Einfluss beliebiger, frei wählbarer Messbedingungen wie z.B. Spannung, Temperatur, Wirkstoffgaben, intrazelluläre oder extrazelluläre Zusammensetzung der Salzlösung, usw.

Ein entscheidender Nachteil der Patch Clamp Methode für den Einsatz in der industriellen Wirkstofffindung ist die aufwendige Vorbereitung der Messungen, die auch erfahrenen Elektrophysiologen nur etwa 20 Messungen pro Tag erlaubt. Zudem erfordert die konventionelle Patch-Clamp-Technik große Erfahrung und viel Fingerspitzengefühl. Verschiedene Arbeitsgruppen und Firmen arbeiten daher daran, die Patch Clamp Methode oder eine vergleichbare andere Messanordnung zu automatisieren oder zu parallelisieren.

Aus WO 00/34776, WO 96/13712 und WO 98/50791 sind Konzepte zur Automatisierung der bishengen Patch Clamp Methode mit Glaspipetten bekannt. Hierbei werden einzelne oder alle der aufwendigen manuellen Arbeitsschritte maschinell unter der Steuerung eines Computers durchgeführt. Dieser Ansatz entlastet den Experimentator und erhöht die Anzahl der durchgeführten Messungen um einen gewissen Faktor, jedoch in der Regel nicht mehr als zehnfach. Diese Art der Automatisierung ist darüber hinaus jedoch technisch sehr anspruchsvoll und benötigt eine umfangreiche Geräteausstattung und ist auch nicht in hinreichendem Maße parallelisierbar.

Aus WO 98/22819, WO 01/27614, WO 99/66329, WO 01/25769, DE 199 36 302, EP 0 962 524, EP 0 960 933, WO 99/28037, WO 99/31503, WO 01/48474, WO 01/48475, WO 01/59447 sind weitere Verfahren und Vorrichtungen zur Automatisierung der Patch-Clamp-Technik bekannt, bei denen die Patch Clamp Pipette durch einen planaren oder einen mikrostrukturierten Träger mit einer geeigneten Öffnung ersetzt wird. Zum Beispiel kann es sich dabei um eine Membran oder dünne Folie handeln, die mit wenige Mikrometer großen Löchern versehen worden ist. Die beschriebenen Verfahren und Vorrichtungen beruhen nun darauf, dass Zellen sich an die Löcher anlagern und sich dort eine Abdichtung ähnlich des Gigaseals bei der Patch Pipette bildet. Dieses Anlagern der Zellen an die Löcher kann durch zufällige Bewegung der Zellen geschehen, oder es kann durch einen Druckgradienten, durch inhomogene elektrische Felder, mit sogenannten optischen Pinzetten oder durch sonstige Kräfte auf die Zelle induziert oder unterstützt werden. Nach erfolgreicher Anlagerung einer Zelle an eine Apertur, so dass die Apertur durch die Membran der Zelle elektrisch dicht verschlossen ist, wird durch das Öffnen der Zellmembran, das chemisch, durch Druck oder elektrisch erfolgen kann, ein elektrisch leitender Zugang durch die Apertur in das Innere der Zelle erzielt. So erhält man in den beschriebenen Verfahren und Vorrichtungen die benötigte elektrisch leitende Verbindung zum Innern der Zelle und kann nachfolgend elektrophysiologische Messungen durchführen. Durch die planare Anordnung und die grundsätzliche Möglichkeit, in einem Träger mehrere Löcher parallel mit Zellen zu belegen, lässt sich der Durchsatz dieser Vorrichtungen und Verfahren steigern. Entscheidendes Merkmal all dieser Verfahren und Vorrichtungen ist, dass die Träger eine oder mehrere geeignete Aperturen aufweisen und die Zellen für die Messung präzise auf der Apertur platziert werden müssen.

Beide beschriebenen Konzepte zur Automatisierung der Patch-Clamp-Technik funktionieren nur mit Zellen, die sich in Suspension befinden. Bei adhärent wachsenden Zelllinien sind die Zellen zunächst vom Träger abzulösen. Dies kann entweder mechanisch, durch geeignete Salzlösungen (z.B. Calzium freie) oder über Enzyme erfolgen und führt in der Regel zu einer starken Belastung der Zellen.

Stand der Technik bei der Materialbearbeitung mit Laserstrahlen sind Laserablationsverfahren, bei denen die gewünschten Strukturen in den zu strukturierenden Materialien mittels intensiver in der Regel stark fokussierter Laserstrahlen, bevorzugt gepulsten Lasern oder Kurzpulslasern erzeugt werden. Durch Fokussierung der Laserstrahlen lassen sich im Fokus Intensitäten im Bereich von Gigawatt und sogar Terawatt pro Quadratzentimeter erzielen, so dass chemische Bindungen aufgebrochen werden. Als Laser kommen sowohl kontinuierliche als auch gepulste Lichtquellen im UV-Bereich, z.B. Excimerlaser, Stickstofflaser als auch Kurzpulslaser und Ultrakurzpulslaser im Sichtbaren und im nahen Infrarot, insbesondere Titan-Saphir-Laser, zum Einsatz. Die typischen Strukturgrößen, die sich mit diesen Verfahren erzielen lassen, hängen von der Wellenlänge, dem verwendeten Laser, den Materialeigenschaften und dem Fokusdurchmesser ab. Aus P. Simon et al. (Photonics Sci News 5, 59 (1999)) und S. Nolte et al (Nonlinear Opt. **24**, 229 (2001)) ist bekannt, dass sich durch Laserablation Strukturen mit einer lateralen Ausdehnung von weniger als einen Mikrometer herstellen lassen, die ein hohes Aspektverhältnis aufweisen, wobei das Aspektverhältnis durch das Verhältnis zwischen der Höhe und der Breite der Struktur gegeben ist.

Bekannter Stand der Technik ist außerdem, dass sich mit diesen Laserablationsverfahren auch biologische Materialien und lebende Gewebe, insbesondere auch Zellen und deren Zellmembranen, bearbeiten lassen. Neben VUV und UV-Laser lassen sich auch hier Kurzpulslaser im nahen Infrarot einsetzen. Bei ausreichend hohen Intensitäten von Gigawatt bevorzugt Terawatt pro Quadratzentimeter kommt es direkt zu Photoablationsprozessen bei denen molekulare Bindungen aufgebrochen werden und kein nennenswerter Wärmeeintrag in die Umgebung stattfindet. Das gilt insbesondere bei ultrakurzen Pulsen im Piko- und Femtosekundenbereich. Aus K. König et al. (Opt. Lett. **26**, 819 (2000)) ist bekannt, dass sich mit einem Kurzpulslaser im nahen Infrarot die Chromosomen im Zellkern zerschneiden lassen, ohne die umgebende Zelle zu schädigen. Die minimale Schnittbreite liegt hierbei bei 100 Nanometern.

Ziel der vorliegenden Erfindung ist es, ein Verfahren zur Durchführung elektrischer Messungen an biologischen Membrankörpern bereitzustellen. Dieses Verfahren ist vorzugsweise geeignet, eine große Anzahl von biologischen Membrankörpern zu untersuchen und ist automatisierbar und parallelisierbar. Der für die elektrische Messung benötigte leitende Zugang zum biologischen Membrankörper soll hierbei durch den Träger und den darauf aufliegenden biologischen Membrankörper hindurch verlaufen und in situ, das heißt ohne den biologischen Membrankörper vorher vom Träger abzulösen, mittels eines Laserablationsprozesses erzeugt werden.

Voraussetzung für diese Erfindung ist, dass zu untersuchende biologische Membrankörper sich auf einem Träger, befinden. Geeignete Träger sind alle dünnen Schichten aus einem Material, an dem die biologischen Membrankörper adhärieren können. Geeignete Träger sind z.B. Folien aus Kunststoff, wie z.B. aus Polyimid, Silikon, Polydimethylsiloxan, Polycarbonat, Polymethylmethacrylat (PMMA), Polytetrafluorethylen (PTFE), ABS (Acyrlnitril / Butadien / Styrol), Polyamid (PA), Polyethylen (PE), Polyethylenterephthalat (PET), Polybuylenterephthalat (PBT), Polypropylen, Polystyrol oder Träger die anorganischen Materialen, wie zum Beispiel Borsilikat-Dünnglas, Quarzglas, Silizium, Siliziumoxid, Siliziumnitrit, Aluminiumoxid, CVD-Diamant oder Keramik enthalten. Bevorzugt werden nicht leitende Materialien verwendet, die eine Elektrizitätskonstante von weniger als ε = 20, bevorzugt von weniger als ε = 10, aufweisen. Die Träger weisen eine Dicke zwischen 10 µm und 500 µm, bevorzugt zwischen 30 und 200 µm. Gegebenfalls ist die Oberfläche des Trägers für die Adhäsion von Zellen oder anderen biologischen Membrankörpern mit einer geeigneten Beschichtung zu versehen, die das Anhaften der zu untersuchenden Zellen oder anderer biologischer Membrankörper begünstigt. Für diesen Zweck sind eine ganze Reihe von Beschichtungen in der Literatur beschrieben worden. Dazu gehören zum Beispiel Proteine wie Collagen, Laminin oder Cadherin, Antikörper, synthetisches Polylysin, Lectine wie z.B. Concanavalin A, oder Polysaccharide wie z.B. Chitosan. Alternativ dazu ist die Oberfläche des Trägers durch eine Plasmabehandlung, zum Beispiel in einem Argon-Plasma zu hydrophilisieren, so dass sie attraktiv für biologische Membranen ist.

Biologische Membrankörper, im Sinne der Erfindung, sind zum Beispiel lebende Zellen. Dazu gehören Zellen, die aus lebenden Geweben durch Dissoziation isoliert wurden (Primärkulturen). Dazu gehören ebenso Zellen, die als etablierte Zelllinien in Kultur gehalten werden, wie CHO-Zellen, HEK-Zellen, NIH3T3-Zellen, HeLa-Zellen aber auch transient transfizierte Zellen oder Primärzellen. Biologische Membrankörper, im Sinne der Erfindung sind außerdem künstlich erzeugte Membrankörper, bei welchen z.B. eine Lipiddoppelschicht ein begrenztes Volumen eines wässrigen Mediums einschließt (Vesikel). Diese Membrankörper enthalten dann vorzugsweise mindestens eine biologische Komponente, z.B. ein in der Lipiddoppelschicht eingelagertes Polypeptid, ein membranständiges Enzym, einen Ionenkanal oder einen G-Protein gekoppelten Rezeptor. Biologische Membrankörper, im Sinne der Erfindung können auch bakterielle Zellen, Pilzzellen oder Zellen anderer einzelliger oder mehrzelliger Organismen sein. Biologische Membrankörper, im Sinne der Erfindung sind, z.B. auch Protoplasten von Pilzzellen und Pflanzenzellen sowie biologische Membrankörper, die - wie z.B. Synaptosomen - durch Abspaltung oder Vereinigung aus den Membranen von lebenden Organismen erzeugt oder die durch die Vereinigung solcher Präparate mit synthetischen Lipidvesikeln erhalten worden sind.

Ein Bereich des Trägers mit mindestens einem darauf liegenden biologischen Membrankörper wird in einem optischen Gerät, z.B. einem inversen oder aufrechten Mikroskop, einem konfokalen Laserscanning-Mikroskop oder einem anderen optischen Gerät, in dem die biologischen Membrankörper einzeln betrachtet beziehungsweise identifiziert werden können, so positioniert, dass ein gewünschter biologischer Membrankörper mittig bezüglich der optischen Achse des Beleuchtungsstrahlengangs zu liegen kommt. Durch eine geeignete Optik wie beispielsweise eine Faserkopplung wird nun gebündelte elektromagnetische Strahlung, z.B. ein Laserstrahl so in das optische Gerät eingekoppelt, dass sich der Fokus der Strahlung auf Trägerunterseite, also der dem biologischen Membrankörper abgewandten Seite befindet.

In einem Fall der vorliegenden Erfindung wird der Laserstrahl hierbei durch eine geeignete Optik, zum Beispiel ein Mikroskop-Objektiv mit hoher numerischer Apertur, von unten auf die Trägerunterseite fokussiert. Bei diesem Laser handelt es sich um einen leistungsstarken Laser, bevorzugt ein Pulslaser, besonderes bevorzugt einen UV Pulslaser oder einen Kurzpulslaser, besonders bevorzugt einen Ultrakurzpulslaser im nahen Infrarot. Durch die Fokussierung dieses Laserstrahls wird auf der Unterseite des Trägers im Fokus eine Intensität von mehr als einem Gigawatt pro Quadratzentimeter, bevorzugt Terawatt pro Quadratzentimeter erzielt.

In einem anderen Fall der vorliegenden Erfindung wird der Laserstrahl von oben durch den biologischen Membrankörper und den Träger hindurch auf die Unterseite des Trägers fokussiert. Bei diesem Laser handelt es sich um einen leistungsstarken Laser, bevorzugt einen Pulslaser oder einen Kurzpulslaser im nahen Infrarot, so dass eine Wechselwirkung des Laserstrahls mit dem Träger und dem biologischen Membrankörper nur bei den sehr hohen Intensitäten im Fokus des Laserstrahls stattfindet. Bei der Wechselwirkung handelt es sich vorzugsweise um Mehrphotonenprozesse, also Prozesse, die nur dann auftreten, wenn zwei oder mehrere Photonen gleichzeitig mit dem biologischen Membrankörper und dem Träger wechselwirken. Aus R. Williams et al. (Curr. Opin. Chem. Biol. 5, 603 (2001)) ist bekannt, dass außerhalb des Fokus die Intensität des Laserstrahls so gering ist, dass keine Mehrphotonenprozesse auftreten und kaum oder keine Wechselwirkung zwischen Laserstrahl und Materie stattfindet.

In beiden beschriebenen Fällen der vorliegenden Erfindung wird nun der Träger durch den Laserstrahl in der Weise strukturiert, dass ein für elektrische Messungen geeigneter Zugang zum Inneren des biologischen Membrankörpers gebildet wird. Hierbei wird der Träger beginnend an der Unterseite, dass heißt der dem biologischen Membrankörper abgewandten Seite, in einem eng begrenzten Raumbereich durch den Laser abgetragen. Bei der Abtragung handelt es sich um einen Laser-Ablationsprozess, der einen Kanal, bevorzugt mit trapezförmigen oder trapezähnlichem Querschnitt im Träger erzeugt, wobei der Kanal auf der Unterseite des Trägers relativ breit beginnt und dann schräg zusammenläuft, so dass besagter Kanal an der besagtem biologischen Membrankörper zugewandten Trägerseite einen Durchmesser von vorzugsweise mindestens 200 nm und höchstens 10 µm, bevorzugt aber einen Durchmesser von mindestens 500 nm und höchstens 5 µm, ganz besonders bevorzugt aber einen Durchmesser von mindestens 1 µm und höchstens 5 µm hat.

An der Trägerunterseite weist der Kanal einen Durchmesser von 10 Mikrometern bis zu einem Millimeter, bevorzugt 20 Mikrometer bis 200 Mikrometer auf. Der Kanal endet zunächst 10 bis 500 Nanometer, bevorzugt 100 bis 200 Nanometer unterhalb der Zelloberfläche. Um eine entsprechende Aufweitung des Kanals auf der Trägerunterseite zu erreichen, muss gegebenenfalls der Träger inklusive des betrachteten biologischen Membrankörpers durch geeignete Mikromanipulatoren bewegt werden. Alternativ kann man auch den Laserstrahl über Ablenkspiegel oder andere geeignete Ablenkeinheiten bewegen. Besonders geeignet sind hierfür konfokale Laserscanning-Mikroskope, da hier die entsprechenden Ablenkeinheiten bereits im Mikroskop integriert sind.

Zur Beschleunigung oder zur Erhöhung der Präzision des Ablationsprozesses kann der Träger mit geeigneten Absorbern dotiert werden, wobei die Absorber an die Wellenlänge des verwendeten Laserstrahls anzupassen sind. Um die Strukturierungsgeschwindigkeit des Ablationsprozesses zu kontrollieren, kann die Absorberkonzentration auch in Form eines graduellen Übergangs mit hoher Absorberkonzentration an der Trägerunterseite und niedriger Absorberkonzentration an der Trägeroberseite variiert werden. Ferner lässt sich der Strukturierungsprozess dadurch beschleunigen, dass man Pulslaser mit einer hohen Repetitionsrate, bevorzugt mit einer Repetitionsrate im Kilohertz-Bereich und besonders bevorzugt mit einer Repetitionsrate im Megahertz-Bereich einsetzt.

Bei Wahl der Laser und Laserparameter wird darauf geachtet wurde, dass nur ein sehr geringer Wärmeeintrag in das umgebende Material stattfindet. Dies erreicht man bevorzugt dadurch dass man Kurzpulslaser mit sehr kurzen Pulsen, bevorzugt mit Pulsen kürzer als eine Nanosekunde und besonders bevorzugt mit Pulsen kürzer als eine Picosekunde einsetzt.

In einem Fall der vorliegenden Erfindung zeichnet sich der Kanal in dem Träger dadurch aus, dass die Innenwände glatt sind.

Nach Ausbildung des einseitig offenen Kanals wird der Träger auf der unteren, dem biologischen Membrankörper abgewandten Seite mit einem Elektrodensystem kontaktiert. Insbesondere wird der einseitig offene Kanal mit einer geeigneten Elektrolytlösung befüllt, die mit der Zusammensetzung des Inneren des biologischen Membrankörpers und mit den angestrebten Zwecken des Versuchs kompatibel ist (intrazelluläre Lösung).

Nach der Kontaktierung schließt der Behälter, der das Elektrodensystem umgibt, zur Trägerunterseite elektrisch dicht ab, wobei die Trägerunterseite unter Umständen geeignet zu beschichten ist, beispielsweise mit Silikon, Vaseline oder einem anderen isolierenden Material.

In einem anderen Fall der vorliegenden Erfindung wird die dem biologischen Membrankörper abgewandte Seite zunächst mit dem Elektrodensystem in Kontakt gebracht und anschließend der beschriebene Ablationsprozess durchgeführt.

Für die Durchführung elektrischer Messungen nach Maßgabe dieser Erfindung ist es notwendig, einen geschlossenen Stromkreis herzustellen, wie er auch für klassische Patch-Clamp-Messungen Verwendung findet. Das bedeutet, dass die mit dem Inneren des Kanals verbundene Elektrolytlösung auf der Unterseite des Trägers sowie die auf der Oberseite des Trägers befindliche Elektrolytlösung, die den biologischen Membrankörper umgibt, jeweils mit einem geeigneten elektrischen Kontakt an die Eingangspole eines geeigneten elektrischen Messverstärkers angeschlossen werden, während zwischen diesen beiden Elektrolytlösungen sonst keine elektrische Verbindung besteht außer über den zu messenden biologischen Membrankörper und den zu ihm hinführenden Kanal. Für den elektrischen Kontakt zur Elektrolytlösung benutzt man vorzugsweise Silber / Silberchlorid Elektroden, wie sie in der Literatur schon seit langem beschrieben sind. Die Versuchsanordnung umfasst einen Behälter mit Elektrolytlösung und Elektrode sowie Verfahren für die Druckregulation und die notwendige Elektronik zum Auslesen von Patch-Clamp-Signalen.

Eine Form der erfindungsgemäßen Messanordnung wird vorteilhaft so ausgeführt, dass der Behälter, der das Elektrodensystem umschließt für die verwendete Laserstrahlung hinreichend transparent ist, so dass es möglich ist den Laserstrahl durch das Elektrodensystem hindurch auf die Trägeroberseite zu fokussieren. Mittels eines oder mehrerer Laserpulse kann nun, beispielsweise, die verbleibende Trägerschicht abgetragen werden. Anschließend kann der Sealwiderstand, also der Serienwiderstand zwischen den zwei Elektrolytlösungen auf dem Träger und im Inneren des Kanals, gemessen werden. Gegebenenfalls wird durch Anlegen eines unterstützenden Unterdrucks der notwendige Sealwiderstand im Megaohm oder bevorzugt Gigaohm-Bereich erzeugt. Eine Alternative wäre die Ausübung eines Drucks auf den biologischen Membrankörper durch Einsatz des Laserstrahls als optische Pinzette. Der Nachweis des Widerstandes erfolgt in der Regel elektrisch, so wie es auch für Patch-Clamp-Messungen gemacht wird. Nachfolgend kann nun mittels ein oder mehrerer Laserpulse die Zellmembran, die sich unmittelbar oberhalb der Kanalöffnung im Träger befindet, geöffnet werden.

In einer anderen Form der erfindungsgemäßen Messanordnung wird der Laserstrahl von oben durch den biologischen Membrankörper auf die verbleibende Trägerschicht unmittelbar unterhalb des biologischen Membrankörpers fokussiert und diese mittels eines oder mehrerer Laserpulse abgetragen. Anschließend wird der Sealwiderstand zwischen biologischem Membrankörper und Träger vermessen. Gegebenenfalls wird durch Anlegen eines unterstützenden Unterdrucks der notwendige Sealwiderstand im Megaohm oder bevorzugt Gigaohm-Bereich erzeugt. Eine Alternative wäre die Ausübung eines Drucks auf den biologischen Membrankörper durch Einsatz des Laserstrahls als optische Pinzette. Der Nachweis des Widerstandes erfolgt in der Regel elektrisch, so wie es auch für Patch-Clamp-Messungen gemacht wird. Nachfolgend wird nun mittels eines oder mehrerer Laserpulse die Zellmembran, die sich unmittelbar oberhalb der Kanalöffnung im Träger befindet, geöffnet.

In einer anderen Form der erfindungsgemäßen Messanordnung wird mit dem Abtragen der verbleibenden Trägerschicht unterhalb des biologischen Membrankörpers gleichzeitig die unmittelbar anschließende Membran des biologischen Membrankörpers geöffnet.

Für die nachfolgenden elektrischen Messungen werden in der erfindungsgemäßen Messanordnung Standardkomponenten eines Patch-Clamp-Messaufbaus verwendet. Alle gängigen Methoden für elektrophysiologische Untersuchungen können nun eingesetzt werden. Dazu gehören zum Beispiel die Messung des Membranpotentials des biologischen Membrankörpers, die Voltage-Clamp-Messung, die Current-Clamp-Messung, oder die Messung der Membrankapazität. Es können beliebige für die Untersuchung relevante Substanzen der Badlösung zugegeben und so an die Membran des biologischen Membrankörpers gebracht werden, so dass die Auswirkung auf die elektrischen Eigenschaften des biologischen Membrankörpers, wie bei klassischen Patch-Clamp Versuchen, gemessen werden können. Insbesondere können aber auch durch den Kanal beliebige Substanzen, wie z.B. Botenstoffe, Farbstoffe, Fluoreszenzfarbstoffe, Chelatbildner, oder andere biologisch oder messtechnisch interessante Substanzen in das Innere des biologischen Membrankörpers gebracht werden. Damit sind auch optische Messungen an dem biologischen Membrankörper möglich. Da dieser Kanal bei klassischen Patch Clamp Messungen dem Inneren der Patch Pipette entspricht, das für einen Wechsel von Lösungen nur mit erhöhtem Aufwand zugänglich ist, bietet sich hier ein zusätzlicher Vorteil der erfindungsgemäßen Messanordnung.

Sollte sich der Zugang zum Innern des biologischen Membrankörpers im Verlauf der Messung mit Partikeln zusetzen, so kann der Zugang mittels erneuten Laserpulsen wieder geöffnet werden. Durch den verwendeten Aufbau ist zudem eine gleichzeitige optische Beobachtung der biologischen Membrankörper möglich. Ferner ist das beschriebene Verfahren für alle adhärenten Zellen einsetzbar und ist nicht auf Kulturzelllinien beschränkt.

Die Dauer des gesamten Ablationsprozesses ist vom verwendeten Lasersystem und den Trägermaterialien abhängig. Bei Einsatz eines Kurzpulslasers im nahen Infrarot mit hoher Repetitionsrate lässt sich das erfindungsgemäße Verfahren zur Erzeugung des elektrisch leitenden Zugangs in weniger als einer Sekunde durchführen.

Das erfindungsgemäße Verfahren lässt sich einfach automatisieren. Die biologischen Membrankörper können über eine geeignete Bildverarbeitung ausgewählt und der Träger mit Mikromanipulatoren automatisch geeignet positioniert werden. Geeignete Zellen können über ein optisches Verfahren (bei dem beispielsweise ein Ionenkanal und das Green Fluorescent Protein (GFP) gemeinsam exprimiert werden) identifiziert werden. Dies entspricht der Integration eines Fluorescence Activated Cell Sorters (FACS) in den beschriebenen Aufbau.

Der Ablationsprozess lässt sich durch den Einsatz geeigneter computersteuerbarer Laser automatisieren. Gegebenenfalls kann die Bildverarbeitung den Ablationsprozess steuern. Ein Regelkreis zur Steuerung des Ablationsprozesses ist aber auch unter Verwendung des Patch-Clamp Stromkreises realisierbar.

Die vorliegende Erfindung lässt sich parallelisieren, indem beispielsweise alle Kammern einer Mikrotiterplatte zum Beispiel mit 96, 384 oder 1536 Öffnungen entsprechend präpariert werden, so dass jede einzelne einen oben beschriebenen Versuchsaufbau inklusive eines miniaturisierten Elektrodensystems darstellt. Auf die einzelnen Messkammern wird dann das erfindungsgemäße Verfahren entweder sequentiell oder parallel in Gruppen angewendet. Hierfür kommen entsprechende Strahlteileroptiken und Strahlführungskomponenten zum Einsatz. Für das Auslesen der Signale können unter anderem Vielkanal-Verstärkersysteme wie sie aus der MEA (multi-electrode-array)-Technik oder den Detektoren in der Hochenergiephysik bekannt sind zum Einsatz kommen. Damit ist die Erfindung ausgezeichnet für den Einsatz in der industriellen Wirkstoffsuche mit hohem oder sehr hohem Durchsatz geeignet.

Eine Form der erfindungsgemäßen Messanordnung erlaubt es Kanäle im Träger wieder zu verschließen, beispielsweise durch den Einsatz des Laserstrahls, der den Randbereich des Kanals lokal aufschmilzt. Hierdurch wird es möglich mehrere auf einem Träger benachbarte Membrankörper nacheinander zu untersuchen.

Das erfindungsgemäße Verfahren zeichnet sich gegenüber anderen Verfahren zur Automatisierung elektrophysiologischer Messungen dadurch aus, dass keine Positionierung der biologischen Membrankörper benötigt wird und dass die Kanäle, die für das Verfahren benötigt werden in situ im Träger erzeugt werden.

Die Erfindung betrifft ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird.

Die Erfindung betrifft außerdem ein Verfahren zur Messung elektrischer Signale an biologischen Membrankörpern, dadurch gekennzeichnet, unterhalb von mindestens zwei sich auf dem selben Träger befindlichen biologischen Membrankörpern jeweils ein Kanal erzeugt wird, der Kanal jeweils mit einer elektrolytischen Flüssigkeit gefüllt wird, und somit ein elektrischer Zugang zu besagten Membrankörpern geschaffen wird.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei besagter Träger ein nicht leitendes Material enthält, dessen Dielektrizitätskonstante kleiner ist als ε = 20.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei in dem Membrankörper in dem den Kanal abdeckenden Bereich eine Öffnung erzeugt wird.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei besagter Träger Dünnglas, Silizium, Polyimid, Silikon, Polydimethylsiloxan, Polycarbonat, Polymethylmethacrylat (PMMA), Polytetrafluoethylen (PTFE), ABS (Acyrlnitril/Butadien/Styrol), Polyamid (PA), Polypropylen, Polystyrol, Polyethylen (PE), Polyethylenterephthalat (PET), Polybuylenterephthalat (PBT), Siliziumnitrit, Aluminiumoxid, Siliziumoxid, oder CVD-Diamant enthält.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei der biologische Membrankörper eine Oozyte, eine biologische Zelle, eine Kulturzelle, eine Primärzelle, ein Vesikel, oder ein Fragment einer Lipiddoppelschicht ist.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei besagter Kanal an der besagtem biologischen Membrankörper zugewandten Trägerseite einen Durchmesser von mindestens 200 Nanometer und höchstens 10 Mikrometer hat.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei besagter Kanal einen konischen Querschnitt hat und der engere Querschnitt sich auf der dem biologischen Membrankörper zugewandten Trägerseite befindet.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, wobei besagter Kanal mit einem Laser erzeugt wird.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger mit einem Laser erzeugt wird, der Kanal mit einer elektrolytischen Flüssigkeit gefüllt wird, und somit ein elektrischer Zugang zu besagtem Membrankörper geschaffen wird, wobei besagter Kanal nachfolgend mit einem Laser wieder verschlossen wird.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger mit einem Laser erzeugt wird, der Kanal mit einer elektrolytischen Flüssigkeit gefüllt wird, und somit ein elektrischer Zugang zu besagtem Membrankörper geschaffen wird, wobei der Laserstrahl von der dem biologischen Membrankörper zugewandten Seite auf den Träger trifft.

Die Erfindung betrifft außerdem ein Verfahren zur Messung elektrischer Signale an biologischen Membrankörpern, dadurch gekennzeichnet, dass unterhalb eines sich auf einem geeigneten Träger befindlichen biologischen Membrankörpers ein Kanal in besagtem Träger mit einem Laser erzeugt wird, der Kanal mit einer elektrolytischen Flüssigkeit gefüllt wird, und somit ein elektrischer Zugang zu besagtem Membrankörper geschaffen wird, wobei der Laserstrahl von der dem biologischen Membrankörper abgewandten Seite auf den Träger trifft.

Die Erfindung betrifft außerdem eine Vorrichtung zur Messung von elektrischen Signalen an biologischen Membrankörpern, enthaltend einem Laser, eine geeignete Halterung für den Träger und eine elektrische Messeinrichtung, wobei besagte Vorrichtung geeignet ist, mindestens eines der oben stehenden erfindungsgemäßen Verfahren durchzuführen.

Die Erfindung betrifft außerdem ein Verfahren zur Bestimmung der Aktivität von membranständigen Polypeptiden, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, und anschließend an besagtem Membrankörper durch den Kanal elektrische Messungen vorgenommen werden, wobei das Ergebnis der elektrischen Messung von der Aktivität des membranständigen Polypeptids abhängig ist.

Die Erfindung betrifft außerdem das vorstehende Verfahren, wobei besagtes Polypeptid ein Ionenkanal oder ein G-Protein gekoppelter Rezeptor ist.

Die Erfindung betrifft außerdem ein Verfahren zur Auffindung von potentiellen Wirkstoffen, dadurch gekennzeichnet, dass unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, und anschließend an besagtem Membrankörper, in Anwesenheit von jeweils einer Testsubstanz elektrische Messungen vorgenommen werden, wobei diejenigen Testsubstanzen potentielle Wirkstoffe darstellen, bei denen sich die gemessenen elektrischen Signale bei unterschiedlichen Konzentrationen der Testsubstanz signifikant unterscheiden.

Die Erfindung betrifft außerdem durch oben stehendes Verfahren gefundenen Wirkstoffe, sowie deren Verwendung zur Herstellung von Medikamenten. "Wirkstoffe", im Sinne der Erfindung, sind Substanzen, die eine oder mehrere biologische Aktivitäten oder eigenschaften eines Organismus, einer Zelle oder eines Polypeptids beeinflussen können. Beispielhaft seien hier pharmazeutische Wirkstoffe genannt.

"Testsubstanzen", im Sinne der Erfindung, sind alle Substanzen, die in einem der vorstehenden oder nachstehenden Verfahren verwendet werden, verwendet wurden und/oder verwendet werden können.

"Signifikant", im Sinne der Erfindung, bedeutet: die Messwerte unterscheiden sich um Beträge, die gleich oder größer sind als die statistische Messunsicherheit. Die statistische Messunsicherheit kann durch wiederholtes Messen unter gleichbleibenden Bedingungen ermittelt werden. Sie ist dann gleich der Standardabweichung in dieser Messreihe.

Die Erfindung betrifft außerdem ein Verfahren zum Screening von Substanzbibliotheken nach Inhibitoren oder Aktivatoren eines membranständigen Polypeptids, dadurch gekennzeichnet, dass das oben beschriebene Verfahren zur Bestimmung der Aktivität von membranständigen Polypeptiden an einem besagtes membranständiges Polypeptid enthaltenden biologischen Membrankörper in Anwesenheit der einzelnen Substanzen aus der Substanzbibliothek durchgeführt wird, wobei diejenigen Substanzen potentielle Inhibitoren oder Aktivatoren des membranständigen Polypeptids darstellen, deren Anwesenheit und/oder Konzentration einen signifikanten Einfluss auf die Aktivität des membranständigen Polypeptids hat.

Die Erfindung betrifft außerdem die durch das oben stehende Verfahren gefundenen Aktivatoren oder Inhibitoren, sowie deren Verwendung zur Herstellung von Medikamenten.

### Beschreibung der Zeichnungen

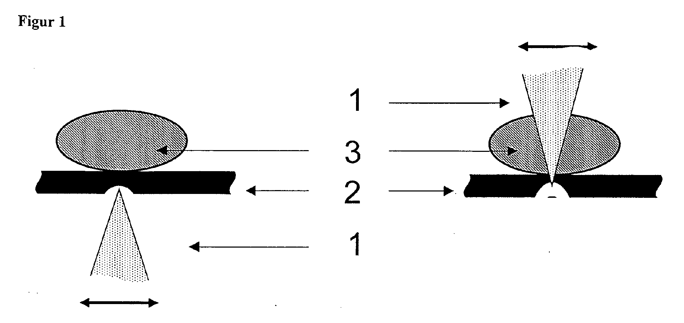

Figur 1 ist eine schematische, nicht maßstabgetreue und nicht detailgenaue Darstellung des Aufbaus für das beschriebene Verfahren, bestehend aus dem Laserstrahl 1, einem Träger 2, und dem biologischen Membrankörper 3. In einem Fall kommt der Laserstrahl 1 von unten (linke Abbildung), im anderen Fall von oben (rechte Abbildung).

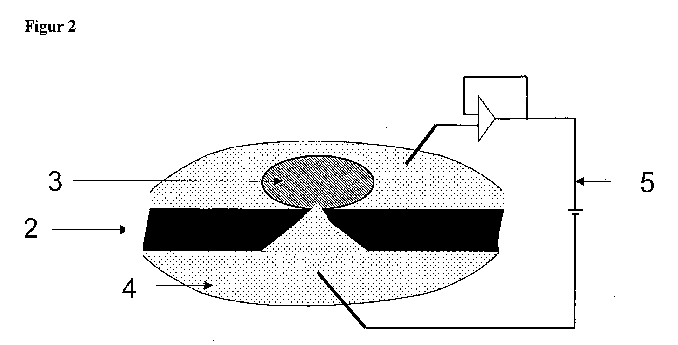

Figur 2 ist eine schematische, nicht maßstabgetreue und nicht detailgenaue Darstellung des Aufbaus für die elektrische Messung, bestehend aus Träger 2, dem biologischen Membrankörper 3 und der elektronischen Schaltung für die elektrische Messung 4.

### Beispiele

Das Verfahren ist speziell geeignet für den Einsatz bei der Wirkstoffsuche, als Ersatz konventioneller und automatisierter Patch-Clamp-Techniken und als portabler Biosensor, beispielsweise in der Umweltanalytik. Beispielhafte Messanordnungen sowie Anwendungsgebiete derselben werden in der Folge beschrieben.

Die erfindungsgemäße Messanordnung weist z.B. mindestens einen Träger auf und auf diesem Träger befinden sich geeignete biologische Membrankörper für elektrische Messungen.

Auf der Unterseite des Trägers befindet sich beispielsweise eine Vorrichtung, die mindestens eine Elektrode enthält und ein für die Aufnahme von Flüssigkeit geeignetes Kompartiment aufweist. Ferner weist die Messanordnung die entsprechende Elektronik und Software zum Auslesen, Darstellen und Auswerten der Messsignale auf.

Gegebenenfalls weist die Messanordnung eine Vorrichtung auf, mit der ein Laserstrahl auf den Träger unterhalb des biologischen Membrankörpers und die Membran unmittelbar oberhalb des Trägers fokussiert werden kann.

Vorzugsweise weist die Messanordnung auf einer Seite oder bevorzugt auf beiden Seiten des Trägers Mittel auf, die eine Flüssigkeitszugabe, eine Flüssigkeitsspeicherung und auch einen Austausch der Flüssigkeiten ermöglichen.

### Beispiel 1

Auf einem Träger aus Borsilikat-Dünnglas (Schott Displayglas D 263 T, Dicke 50 Mikrometer) werden CHO-Zellen kultiviert, so dass die Dichte der Zellen derart ist, dass einzelne isoliert liegende Zellen ohne Mühe identifiziert werden können. Der Träger ist durch eine Erhöhung begrenzt, so dass Trägeroberfläche und Erhöhung ein flaches Gefäß bilden, dass mit einer für die Zellen geeigneten Nähr- oder Elektrolytlösung gefüllt ist.

Der Träger mit adhärenten Zellen wird auf dem Tisch eines aufrechten Laser-Scanning-Mikroskops (Zeiss LSM 510) positioniert. Mittels konventioneller Auflicht-Mikroskopie wird eine geeignet erscheinende Zelle selektiert und mittels des Mikroskoptisches mittig bezüglich der optischen Achse positioniert. Zusätzlich wird eine Elektrode in die Lösung eingebracht, so dass diese mit der Lösung einen elektrischen Kontakt herstellt, den Strahlengang jedoch nicht behindert.

Der Tisch des Mikroskops ist dahingehend modifiziert, dass im Bereich der optischen Achse des Systems auch von unten ein Elektrodensystem mit dem Träger in Kontakt gebracht werden kann. Hierbei handelt es sich um ein kleines Gefäß, das mit Elektrolytlösung gefüllt ist und in dem sich eine Elektrode befindet. Die nach oben gewandte Seite des Gefäßes ist offen, die andere Seite ist geschlossen oder an ein geeignetes System zur Einstellung des Druckes sowie für den Austausch von Lösungen oder für die Zugabe von Substanzen angeschlossen. Die Elektrode wird mit dem Verstärker und der Messelektronik verbunden. Die Seite des Gefäßes, die nach oben zeigt, ist offen und der Rand kann z.B. mit Silikon beschichtet sein, um eine mechanische Abdichtung gegenüber der Unterseite des Trägers zu erzielen.

In das Laser-Scanning-Mikroskop wird ein leistungsstarker Kurzpulslaser im nahen Infrarot (Spectra Physics Mai Tai) eingekoppelt und mittels eines Objektivs mit großem Arbeitsabstand durch die Zelle hindurch auf die Trägerunterseite fokussiert. Innerhalb der Fokusebene ergibt sich für diesen Laser eine Leistung von mehr als 300 Milliwatt. Mit dem Laserstrahl wird nun der Träger beginnend an der Unterseite abgetragen, so dass sich ein Kanal bildet, der an der Unterseite einen Durchmesser von 100 Mikrometern und unmittelbar unterhalb der Zelle einen Durchmesser von einem Mikrometer aufweist. Bevor der Kanal auf der Oberfläche des Trägers austritt wird das Elektrodensystem in Kontakt mit der Unterseite des Trägers gebracht, so dass sich beim Kontakt von Zylinder und Unterseite des Glasträgers eine elektrisch dichte Verbindung ausbildet. Mit einem oder mehreren Laserpulse wird nun das restliche Trägermaterial unterhalb der Zelle abgetragen, so dass die Zellmembran einen direkten Kontakt zum Elektrodensystem bekommt.

Für intrazelluläre Messungen wird nun die Zellmembran mit einem oder mehreren Laserpulsen im Bereich der Kanalöffnung geöffnet und dieses über die entsprechenden elektronischen Signale nachgewiesen. Für die nachfolgenden elektrischen Messungen können alle gängigen Methoden für elektrophysiologische Untersuchungen eingesetzt werden. Neben den elektrischen Messungen ist zudem eine gleichzeitige optische Beobachtung der Zellen möglich.

Sollte sich der Zellzugang im Verlauf der Messung mit Partikeln zusetzen, so wird der Zugang mittels einer oder mehrerer Laserpulsen wieder geöffnet.

### Beispiel 2

Auf einem Träger aus Borsilikat-Dünnglas (Schott Displayglas D 263 T, Dicke 50 Mikrometer) werden CHO-Zellen kultiviert, so dass die Dichte der Zellen derart ist, dass einzelne isoliert liegende Zellen ohne Mühe identifiziert werden können. Der Träger ist durch eine Erhöhung begrenzt, so dass Trägeroberfläche und Erhöhung ein flaches Gefäß bilden, dass mit einer für die Zellen geeigneten Nähr- oder Elektrolytlösung gefüllt ist.

Der Träger mit adhärenten Zellen wird auf dem Tisch eines inversen Laser-Scanning-Mikroskops (Zeiss LSM 510) positioniert. Mittels konventioneller Mikroskopie wird dann eine geeignete Zelle selektiert und mittels des Mikroskoptisches mittig bezüglich der optischen Achse positioniert. Zusätzlich wird eine Badelektrode in die Lösung eingebracht, so dass diese sich möglichst nah an der selektierten Zelle befindet.

Der Tisch des Mikroskops ist dahingehend modifiziert, dass im Bereich der optischen Achse des Systems von unten ein Elektrodensystem mit dem Träger in Kontakt gebracht werden kann. Hierbei handelt es sich um einen kleines Gefäß, das mit Elektrolytlösung gefüllt ist und in dem sich eine Elektrode befindet. Die untere Seite des Gefäßes ist mit einer Glasplatte mit optischer Qualität verschlossen die transparent für die verwendete Laserstrahlung ist, so dass der Laserstrahl durch das Elektrodensystem hindurch auf das Trägermaterial fokussiert werden kann. Die seitliche Öffnung des Gefäßes kann mit einem geeignetes System zur Einstellung des Druckes sowie für den Austausch von Lösungen oder für die Zugabe von Substanzen verbunden werden oder ist verschlossen. Die Elektrode wird mit dem Verstärker und der Messelektronik verbunden. Die obere Seite des Gefäßes ist offen und der Rand kann z.B. mit Silikon beschichtet sein, um eine mechanische Abdichtung gegenüber der Unterseite des Trägers zu erzielen.

In das Laser-Scanning-Mikroskop wird ein leistungsstarker Kurzpulslaser im nahen Infrarot (Spectra Physics Mai Tai) eingekoppelt und mittels eines Mikroskop-Objektivs auf die Trägerunterseite fokussiert. Innerhalb der Fokusebene ergibt sich für diesen Laser eine Leistung von mehr als 300 Milliwatt. Mit dem Laserstrahl wird nun der Träger beginnend an der Unterseite abgetragen, so dass sich ein Kanal bildet, der an der Unterseite einen Durchmesser von 100 Mikrometern und unmittelbar unterhalb der Zelle einen Durchmesser von einem Mikrometer aufweist. Unmittelbar bevor der Kanal auf der Oberfläche des Trägers austritt wird das Elektrodensystem in Kontakt mit der Unterseite des Trägers gebracht, so dass sich beim Kontakt von Zylinder und Unterseite des Glasträgers eine elektrisch dichte Verbindung ausbildet. Das Objektiv wird gegen ein Objektiv mit längerem Arbeitsabstand ausgetauscht und der Laserstrahl mit diesem durch das Elektrodensystem hindurch auf die Trägeroberseite fokussiert. Mit einem oder mehreren Laserpulse wird nun das restliche Trägermaterial unterhalb der Zelle abgetragen, so dass die Zellmembran einen direkten Kontakt zum Elektrodensystem bekommt.

Für intrazelluläre Messungen wird nun die Zellmembran mit einem oder mehreren Laserpulsen im Bereich der Kanalöffnung geöffnet und dieses über die entsprechenden elektronischen Signale nachgewiesen. Für die nachfolgenden elektrophysiologischen Messungen können alle gängigen Methoden für elektrophysiologische Untersuchungen eingesetzt werden. Neben den elektrophysiologischen Messungen ist zudem eine gleichzeitige optische Beobachtung der Zellen von oben möglich.

Sollte sich der Zellzugang im Verlauf der Messung mit Partikeln zusetzen, so wird der Zugang mittels einer oder mehrerer Laserpulsen wieder geöffnet werden.

### Beispiel 3

Wie Beispiel 2, wobei in das Laser-Scanning-Mikroskop ein leistungsstarker UV-Kurzpulslaser eingekoppelt und mittels eines Mikroskop-Objektivs auf die Trägerunterseite fokussiert wird.

### Beispiel 4

Entsprechend den Beispielen 1 bis 3, wobei Schott Displayglas AF 45, 50 Mikrometer stark, verwendet wird.

### Beispiel 5

Entsprechend den Beispielen 1 bis 4, wobei das Elektrodensystem vor dem Beginn des Ablationsprozesses mit dem Träger in Kontakt gebracht wird.

### Beispiel 6

Entsprechend den Beispielen 1 bis 4, wobei die Zellmembran im Bereich der Kanalöffnung nicht geöffnet wird.

## Patentansprüche

1. Verfahren zur Messung elektrischer Signale an biologischen Membrankörpern, **dadurch gekennzeichnet, dass** unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird.

2. Verfahren nach Anspruch 1, bei dem unterhalb von mindestens zwei sich auf dem selben Träger befindlichen biologischen Membrankörpern jeweils ein Kanal erzeugt wird.

3. Verfahren nach Anspruch 1, bei dem besagter Träger ein elektrisch nicht leitendes Material enthält, dessen Dielektrizitätskonstante kleiner ist als ε = 20.

4. Verfahren nach Anspruch 1, bei dem in dem Membrankörper in dem den Kanal abdeckenden Bereich eine Öffnung erzeugt wird.

5. Verfahren nach Anspruch 1, bei dem besagter Träger Dünnglas, Silizium, Polyimid, Silikon, Polydimethylsiloxan, Polycarbonat, Polymethylmethacrylat (PMMA), Polytetrafluoethylen (PTFE), ABS (Acyrlnitril/Butadien/Styrol), Polyamid (PA), Polypropylen, Polystyrol, Polyethylen (PE), Polyethylenterephthalat (PET), Polybuylenterephthalat (PBT), Siliziumnitrit, Aluminiumoxid, Siliziumoxid, oder CVD-Diamant enthält.

6. Verfahren nach Anspruch 1, bei dem der biologische Membrankörper
i) eine Oozyte,
ii) eine biologische Zelle,
iii) eine Kulturzelle,
iv) eine Primärzelle,
v) ein Vesikel, oder
vi) ein Fragment einer Lipiddoppelschicht
ist.

7. Verfahren nach Anspruch 1, bei dem besagter Kanal an der besagtem biologischen Membrankörper zugewandten Trägerseite einen Durchmesser von mindestens 200 nm und höchstens 10 µm hat.

8. Verfahren nach Anspruch 1, bei dem besagter Kanal einen konischen Querschnitt hat wobei der engere Querschnitt sich auf der dem biologischen Membrankörper zugewandten Trägerseite befindet.

9. Verfahren nach Anspruch 1, bei dem besagter Kanal mit einem Laser erzeugt wird.

10. Verfahren zur Messung elektrischer Signale an biologischen Membrankörpern, bei dem zunächst die Schritte des Verfahrens gemäß Anspruch 9 ausgeführt werden, und bei dem nachfolgend besagter Kanal mit einem Laser wieder verschlossen wird.

11. Verfahren nach Anspruch 9, bei dem der Laserstrahl von der dem biologischen Membrankörper zugewandten Seite auf den Träger trifft.

12. Verfahren nach Anspruch 9, bei dem der Laserstrahl von der dem biologischen Membrankörper abgewandten Seite auf den Träger trifft.

13. Vorrichtung zur Messung von elektrischen Signalen an biologischen Membrankörpern, enthaltend
i) einem Laser,
ii) eine geeignete Halterung für den Träger,
iii) und eine elektrische Messeinrichtung,
wobei besagte Vorrichtung geeignet ist, mindestens eines der Verfahren gemäß Anspruch 1 bis 12 durchzuführen.

14. Verfahren zur Bestimmung der Aktivität von membranständigen Polypeptiden, **dadurch gekennzeichnet, dass** unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, und anschließend an besagtem Membrankörper durch den Kanal elektrische Messungen vorgenommen werden, wobei das Ergebnis der elektrischen Messung von der Aktivität des membranständigen Polypeptids abhängig ist.

15. Verfahren nach Anspruch 14, wobei besagtes Polypeptid ein Ionenkanal oder ein G-Protein gekoppelter Rezeptor ist.

16. Verfahren zur Auffindung von Wirkstoffen, **dadurch gekennzeichnet, dass** unterhalb eines sich auf einem geeigneten Träger befindlichen biologischen Membrankörpers ein Kanal in besagtem Träger erzeugt wird, der Kanal mit einer elektrolytischen Flüssigkeit gefüllt wird, und somit ein elektrischer Zugang zu besagtem Membrankörper geschaffen wird, und anschließend an besagtem Membrankörper, in Anwesenheit jeweils einer Testsubstanz elektrische Messungen vorgenommen werden, wobei diejenigen Testsubstanzen Wirkstoffe darstellen, bei denen sich die gemessenen elektrischen Signale bei unterschiedlichen Konzentrationen der Testsubstanz signifikant unterscheiden.

17. Durch ein Verfahren gemäß Anspruch 16 gefundene Wirkstoffe.

18. Verwendung eines durch ein Verfahren gemäß Anspruch 16 gefundenen Wirkstoffs zur Herstellung eines Medikaments.

19. Verfahren zum Screening von Substanzbibliotheken nach Inhibitoren oder Aktivatoren eines membranständigen Polypeptids, **dadurch gekennzeichnet, dass** ein Verfahren gemäß Anspruch 14 an einem besagtes membranständiges Polypeptid enthaltenden biologischen Membrankörper in Anwesenheit je einer einzelnen Substanz aus der Substanzbibliothek durchgeführt wird, wobei diejenigen Substanzen potentielle Inhibitoren oder Aktivatoren des membranständigen Polypeptids darstellen, deren Anwesenheit und/oder Konzentration einen signifikanten Einfluss auf die Aktivität des membranständigen Polypeptids hat.

20. Durch ein Verfahren gemäß Anspruch 19 gefundene Inhibitoren oder Aktivatoren eines membranständigen Polypeptids.

21. Verwendung eines durch ein Verfahren gemäß Anspruch 19 gefundenen Inhibitors oder Aktivators eines membranständigen Polypeptids zur Herstellung eines Medikaments.
